# EUROPÄISCHE PATENTSCHRIFT

(11) **EP 0 669 326 B1**
(45) Veröffentlichungstag und Bekanntmachung des Hinweises auf die Patenterteilung: **22.12.1999**
(21) Anmeldenummer: 95102145.0
(22) Anmeldetag: 16.02.1995
(51) Int. Cl.: C07D 261/18, A01N 43/80

(54) **Cyanoisoxazole**
Cyanoisoxazoles
Cyanoisoxazoles

(30) Priorität: 25.02.1994 DE 4406209
(43) Veröffentlichungstag der Anmeldung: 30.08.1995
(73) Patentinhaber: BASF Aktiengesellschaft, 67063 Ludwigshafen (DE)
(72) Erfinder: Münster, Peter, Dr., D-68809 Neulussheim (DE); Ditrich, Klaus, Dr., D-67161 Gönnheim (DE); Hamprecht, Gerhard, Dr., D-69469 Weinheim (DE); Gerber, Matthias, Dr., D-67117 Limburgerhof (DE); Westphalen, Karl-Otto, Dr., D-67346 Speyer (DE); Walter, Helmut, Dr., D-67283 Obrigheim (DE); Karl, Rudolf, Dr., D-67112 Mutterstadt (DE)

(56) Entgegenhaltungen:
- EP-A- 0 002 881
- EP-A- 0 337 263
- EP-A- 0 418 667
- EP-A- 0 503 410
- WO-A-92/16514
- JOURNAL OF THE CHEMICAL SOCIETY, PERKIN TRANSACTIONS I, Nr. 7, Juli 1982 Seiten 2391-2394, A. CAMPARINI ET AL. 'Syntheses and Reactivities of 3-Methylisoxazolo[4,5-b]pyridines'
- JOURNAL OF HETEROCYCLIC CHEMISTRY, Bd. 22,Nr. 5, 1985 Seiten 1561-1565, A. CAMPARINI ET AL. 'Synthesis and Photochemical Reactivity of 3-Methylisoxazolo[4,5-d]pyridazines'
- ZEITSCHRIFT FÜR CHEMIE, Bd. 22,Nr. 4, April 1982 LEIPZIG, DD, Seiten 138-139, G. WESTPHAL ET AL. 'Zur Reaktion von 1,2,5-Oxadiazol-N-oxiden mit Acetylen-dicarbonsäurediethylester'

## Beschreibung

Die vorliegende Erfindung betrifft Cyanoisoxazole der allgemeinen Formel Ia und Ib, 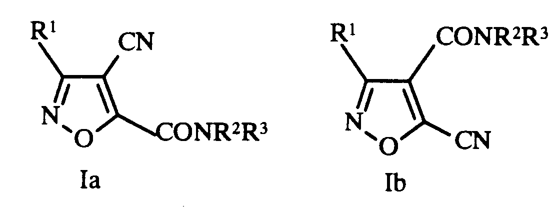 in denen die Substituenten folgende Bedeutung haben:
R¹
Wasserstoff;
C₁-C₆-Alkyl, welches ein bis fünf Halogenatome und/oder einen Cyanorest und/oder bis zu zwei der folgenden Reste tragen kann: C₁-C₄-Alkoxy, C₁-C₄-Halogenalkoxy, C₁-C₄-Alkylthio, C₁-C₄-Halogenalkylthio, C₃-C₆-Cycloalkyl, welches wiederum durch ein bis drei Halogen- oder C₁-C₄-Alkylreste substituiert sein kann oder Phenyl, welches noch einen bis drei der folgenden Reste tragen kann: Cyano, Halogen, Nitro, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio;
C₃-C₈-Cycloalkyl, welches ein- bis dreimal durch C₁-C₄-Alkyl und/oder Halogen substituiert sein kann;
Phenyl, welches noch einen bis drei der folgenden Reste tragen kann: Cyano, Nitro, Halogen, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio;
R²
Wasserstoff (besonders bevorzugt);
C₁-C₆-Alkyl, das einen bis drei der folgenden Substituenten tragen kann: Hydroxy, Halogen, C₁-C₄-Alkoxy und/oder C₁-C₄-Alkylthio;
C₃-C₈-Cycloalkyl, das ein- bis dreimal durch Halogen, C₁-C₄-Alkyl und/oder C₁-C₄-Halogenalkyl substituiert sein kann;
R³
eine C₁-C₄-Alkoxygruppe;
eine C₁-C₆-Alkylgruppe, die einen bis drei der folgenden Reste tragen kann: Halogen, Cyano, C₁-C₄-Alkoxy, C₁-C₄-Halogenalkoxy, C₁-C₄-Alkylthio, C₁-C₄-Halogenalkylthio, C₃-C₈-Cycloalkyl oder Phenyl, wobei der Phenylring seinerseits einen bis drei der folgenden Reste tragen kann: Halogen, Cyano, Nitro, C₁-C₄-Alkyl, C₁-C₄-Halogenalkyl, C₁-C₄-Alkoxy, C₁-C₄-Halogenalkoxy, C₁-C₄-Alkylthio und/oder C₁-C₄-Halogenalkylthio;
eine C₃-C₆-Cycloalkenylgruppe, die ein- bis dreimal durch Halogen oder C₁-C₄-Alkyl substituiert sein kann;
eine C₃-C₈-Cycloalkylgruppe, die einen bis drei der folgenden Reste tragen kann: Halogen, Nitro, Cyano, C₁-C₆-Alkyl, C₁-C₆-Halogenalkyl, C₁-C₄-Alkoxy und/oder C₁-C₄-Halogenalkoxy;
eine C₃-C₆-Alkenyl- oder C₃-C₆-Alkinylgruppe, die jeweils ein- bis dreimal durch Halogen und/oder einmal durch Phenyl substituiert sein können, wobei der Phenylrest seinerseits eine bis drei der folgenden Gruppen tragen kann: C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, Halogen, Cyano oder Nitro;
die Phenylgruppe, welche noch einen bis drei der folgenden Reste tragen kann: Cyano, Nitro, Halogen, C₁-C₆-Alkyl, C₁-C₆-Halogenalkyl, C₁-C₆-Alkoxy, C₁-C₆-Halogenalkoxy, C₁-C₆-Alkylthio und/oder C₁-C₆-Halogenalkylthio.

Außerdem betrifft die Erfindung herbizide Mittel, welche die Verbindungen Ia und/oder Ib als wirksame Substanzen enthalten und Verfahren zur Herstellung dieser Substanzen.

Herbizid wirksame Isoxazolcarbonsäureamide sind bekannt, z.B. aus EP-A-337 263, EP-A-418 667, EP-A-503 410 und WO 92/16514. Trotz der an sich guten herbiziden Wirksamkeit dieser Verbindungen bestand die Aufgabe, ähnliche Verbindungen mit verbesserten ökobiologischen Eigenschaften, insbesondere im Vergleich zu den in oben zitierten Schriften offenbarten freien Carbonsäuren lipophilere Verbindungen zu synthetisieren.

Demgemäß wurden die eingangs definierten Cyanoisoxazole Ia und Ib gefunden.

Die erfindungsgemäßen Carbonsäureamide der Formel Ia und Ib sind auf folgenden zwei Wegen herstellbar:

### Weg 1:

Ein Verfahren zur Synthese der erfindungsgemäßen Isoxazolcarbonsäureamide Ia, in denen R¹, R² und R³ die oben genannten Bedeutungen haben, besteht darin, daß man eine Isoxazolcarbonsäure der Formel II in an sich bekannter Weise in ein Carbonsäurehalogenid X oder eine andere aktivierte Form der Carbonsäure überführt (A) und anschließend mit Ammoniak in das Amid III über-führt (B), welches durch Umsetzung mit wasserentziehenden Mitteln in ein Nitril der Formel Ia umgewandelt werden kann (C).

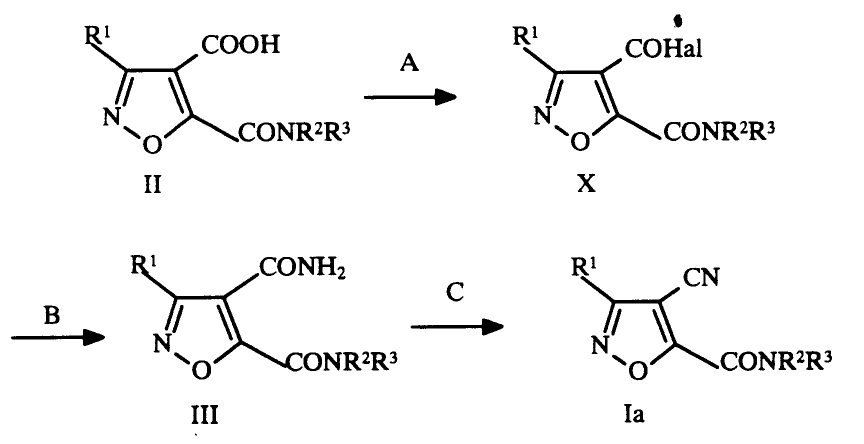

Die für dieses Verfahren benötigten Isoxazolcarbonsäuren sind bekannt.

Aktivierte Formen der Carbonsäure sind neben Halogeniden wie insbesondere den Chloriden und den Bromiden beispielsweise auch Imidazolide. Im allgemeinen werden die Halogenide bevorzugt.

Man erhält sie durch Umsetzung der Carbonsäuren II mit einem Halogenierungsmittel wie Thionylchlorid, Thionylbromid, Phosgen, Phosphoroxychlorid bzw. -bromid, Phosphortri- und -pentachlorid bzw. -bromid sowie elementarem Chlor und Brom.

Das Halogenierungsmittel wird in 1 bis 5 mol-äq., vorzugsweise 1 bis 4 Moläquivalenten, eingesetzt.

Die Umsetzung verläuft bei Temperaturen von 20°C bis zum Siedepunkt des Halogenierungsmittels bzw. sofern man in Gegenwart eines inerten organischen Lösungsmittels arbeitet, auch dessen Siedepunkt.

Als Lösungsmittel eignen sich beispielsweise Kohlenwasserstoffe und Halogenkohlenwasserstoffe wie Benzol, Toluol und Dichlormethan.

Üblicherweise werden die aktivierten Carbonsäurederivate isoliert, beispielsweise durch Abdestillieren des Halogenierungsmittels und sofern vorhanden des Lösungsmittels und erst anschließend mit Ammoniak umgesetzt.

In diesem Fall wird die Amidierung bei Temperaturen von -20 bis 50°C, vorzugsweise 0 bis 30°C in einem inerten aprotisch polaren organischen Lösungsmittel durchgeführt.

Für diese Umsetzung eignen sich insbesondere Halogenkohlenwasserstoffe wie Dichlormethan und Ether wie Diethylether und tert.-Butylmethylether als Lösungsmittel.

Da bei der Amidierung von Säurehalogeniden Halogenwasserstoff gebildet wird, empfiehlt es sich, Ammoniak im Überschuß, bezogen auf den Ausgangsstoff, z.B. in Mengen von 2 bis 5, vorzugsweise 2 bis 3 Moläquivalenten zuzusetzen.

Als wasserentziehende Mittel für den zweiten Reaktionsschritt eignen sich unter anderen literaturbekannten Reagenzien PCl₃, PCl₅, POCl₃, SOCl₂, P₂O₅, Essigsäureanhydrid oder Trifluoressigsäureanhydrid. Die Reaktion kann unverdünnt oder in Lösung durchgeführt werden. Geeignete Lösungsmittel können sein: Ether wie Diethylether, tert.-Butyl-methylether, Tetrahydrofuran oder Dioxan, halogenierte Kohlenwasserstoffe wie insbesondere Methylenchlorid oder 1,2-Dichlorethan, aliphatische oder aromatische Kohlenwasserstoffe oder auch Dimethylformamid.

Die Reaktion kann in einem Temperaturbereich von -20°C bis zur Siedetemperatur der Reaktionslösung durchgeführt werden. Die wasserentziehenden Mittel werden äquimolar oder im Überschuß, bezogen auf das Diamid eingesetzt.

In völlig analoger Weise sind die erfindungsgemäßen Verbindungen Ib gemäß folgendem Reaktionsschema zugänglich.

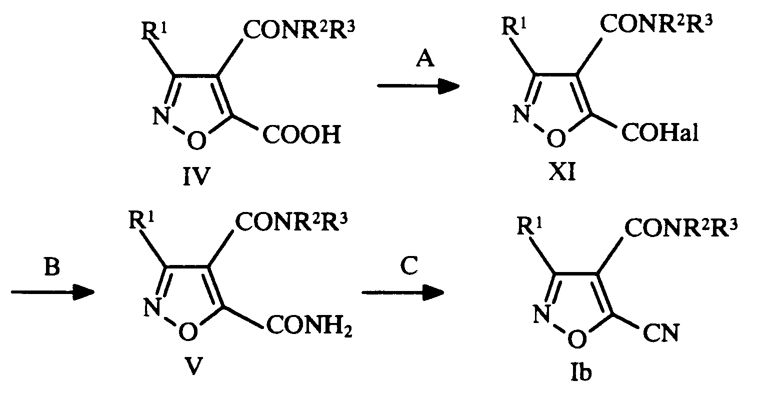

### Weg 2a:

Ein weiteres Verfahren zur Synthese der Verbindungen Ib besteht darin, daß man einen Isoxazol-4,5-dicarbonsäureester der Formel VI in an sich bekannter Weise zum Monoester VII verseift, diesen nach Aktivierung zum Amid VIII umsetzt, anschließend nach einem weiteren Hydrolyseschritt die Säure IX in das Diamid V überführt, welches dann in das Nitril Ib umgewandelt wird.

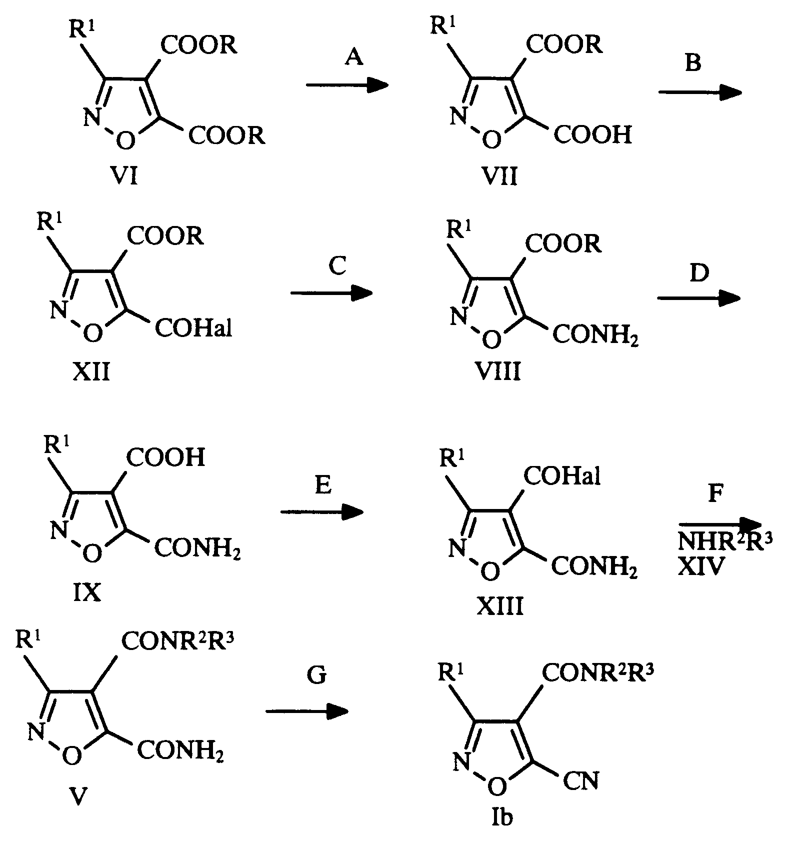

Die Reaktionsschritte B, C und G können analog den vorstehend bei Weg 1 geschilderten Bedingungen durchgeführt werden. Die Umsetzung E erfolgt gemäß den bei Verfahren 1 geschildeten Bedingungen, jedoch vorteilhaft mit der Maßgabe, daß das Aktivierungsmittel, sofern es gleichzeitig auch als wasserentziehendes Mittel benutzt werden kann, nur in einem Verhältnis von 0,95 bis 1,2 Moläquivalenten eingesetzt wird.

Der Reaktionsschritt A wird üblicherweise so durchgeführt, daß man einen Dicarbonsäuredialkylester VI bei Temperaturen zwischen 0 und 80°C, vorzugsweise zwischen 0 und 40°C, in einem organischen Lösungsmittel, z.B. Methanol oder Ethanol, mit einer starken Base, z.B. einem Alkali- oder Erdalkalihydroxid wie NaOH, KOH oder Ca(OH)₂, behandelt. Im allgemeinen wird dabei etwa 1 Äguivalent (z.B. 0,8 bis 1,05 Äq.) der Base in wäßriger Lösung eingesetzt. Nach erfolgter Umsetzung wird abgekühlt und mit einer starken Säure, z.B. einer Mineralsäure wie Salzsäure oder Schwefelsäure, angesäuert. Die entstehende Carbonsäure VII kann auf übliche Art und Weise z.B. durch Absaugen oder durch Extraktion mit einem organischen Lösungsmittel isoliert werden.

Die zweite Esterhydrolyse (Reaktionsschritt D) kann in analoger Weise, vorgenommen werden, wobei es vorteilhaft sein kann, die Base in einem bis zu 5-fachen Überschuß einzusetzen.

Die Carbonsäureamide X erhält man aus den Carbonsäurehalogeniden (Reaktionsschritt F) durch Umsetzung mit einem Amin XIV. Dabei geht man zweckmäßigerweise so vor, daß man das Carbonsäurehalogenid in einem inerten organischen Lösungsmittel wie Dichlorethan, oder einem Ether wie Diethylether oder Methyl-tert.-butylether mit einem Amin XIV, vorteilhaft ebenfalls gelöst in einem organischen Lösungsmittel, zur Reaktion bringt. Dabei setzt man das Amin zweckmäßigerweise in 2- bis 5-fach molarer Menge, vorzugsweise 2- bis 3-fach molarer Menge ein, um den entstehenden Halogenwasserstoff zu binden. Man kann auch in Gegenwart einer Hilfsbase wie einem tertiären Amin, z.B. Triethylamin, arbeiten. In diesem Fall genügen 1 bis 1,5 Moläquivalente Amin XIV. Die Reaktionstemperatur kann zwischen 0 und 50°C, vorzugsweise zwischen 0 und 20°C betragen. Die Reaktion ist im allgemeinen nach 1 bis 12 Stunden beendet. Das Gemisch kann wie üblich aufgearbeitet werden, beispielsweise durch Hydrolyse mit Wasser und Extraktion des Diamids V mit einem organischen Lösungsmittel und Einengen des organischen Lösungsmittels. Zur Reinigung kann das Diamid V beispielsweise umkristallisiert oder chromatographiert werden.

### Weg 2b:

Ein abgewandeltes Verfahren zur Synthese der Verbindungen Ib besteht darin, daß man ein Amid der Formel IX mit einem bis zu 10-fach molaren Überschuß Thionylchlorid in einem inerten organischen Lösungsmittel wie z.B. Toluol oder in Thionylchlorid als Lösungsmittel behandelt. Die Reaktion kann bei Temperaturen von 0°C bis zum Siedepunkt des Gemisches durchgeführt werden. Das so erhaltene Säurechlorid XV kann dann wie vorstehend (F) beschrieben mit einem Amin XIV zu den Produkten Ib umgesetzt werden.

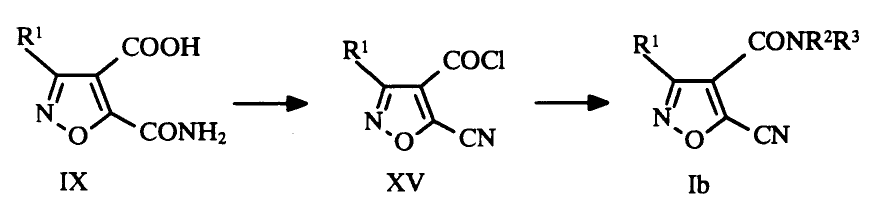

Im Hinblick auf die erfindungsgemäße herbizide Wirkung bei gleichzeitig gutem ökobiologischen Verhalten, z.B. Abbaubarkeit im Boden, haben die Substituenten in den Verbindungen Ia und Ib beispielsweise die folgende Bedeutung:
R¹
Wasserstoff;
unverzweigtes oder verzweigtes C₁-C₆-Alkyl, wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere Methyl, Ethyl, Propyl, 1-Methylethyl und 1,1-Dimethylethyl, welches ein bis fünf Halogenatome wie Fluor, Chlor, Brom, Jod, insbesondere Fluor und Chlor, und/oder einen Cyanorest und/oder bis zu zwei der folgenden Reste tragen kann:
C₁-C₄-Alkoxy wie Methoxy, Ethoxy, Propoxy, 1-Methylethoxy, Butoxy, 1-Methylpropoxy, 2-Methylpropoxy und 1,1-Dimethylethoxy, insbesondere Methoxy, Ethoxy, 1-Methylethoxy und 1,1-Dimethylethoxy;
partiell oder vollständig halogeniertes C₁-C₄-Alkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy und Pentafluorethoxy;
C₁-C₄-Alkylthio wie Methylthio, Ethylthio, Propylthio, 1-Methylethylthio, n-Butylthio, 1-Methylpropylthio, 2-Methylpropylthio und 1,1-Dimethylethylthio, insbesondere Methylthio und Ethylthio;
partiell oder vollständig halogeniertes C₁-C₄-Alkylthio wie Difluormethylthio, Trifluormethylthio, Chlordifluormethylthio, 1-Fluorethylthio, 2-Fluorethylthio, 2,2-Difluorethylthio, 2,2,2-Trifluorethylthio, 2-Chlor-2,2-difluorethylthio, 2,2-Dichlor-2-fluorethylthio, 2,2,2-Trichlorethylthio und Pentafluorethylthio, insbesondere Trifluormethylthio und Pentafluorethylthio;
eine C₃-C₈-Cycloalkylgruppe wie Cyclopropl, Cyclobutyl, Cyclopentyl, Cyclohexyl, Cycloheptyl und Cyclooctyl, insbesondere Cyclopropyl, Cyclopentyl und Cyclohexyl, wobei der Cyclus noch ein-bis dreimal durch C₁-C₄-Alkyl wie vorstehend genannt, insbesondere Methyl und Ethyl; oder Halogen wie Fluor, Chlor, Brom und Jod, insbesondere Fluor und chlor, substituiert sein kann;
die Phenylgruppe, welche noch einen bis drei der folgenden Reste tragen kann: Cyano; Nitro; Halogen wie Fluor, Chlor, Brom und Jod, insbesondere Fluor und Chlor; C₁-C₆-Alkyl wie vorstehend genannt, insbesondere Methyl, Ethyl und 1-Methylethyl; partiell oder vollständig halogeniertes C₁-C₆-Alkyl wie vorstehend genannt, insbesondere Trifluormethyl und Chlordifluormethyl; C₁-C₆-Alkoxy wie vorstehend genannt, insbesondere Methoxy und Ethoxy; partiell oder vollständig halogeniertes C₁-C₆-Alkoxy wie vorstehend genannt, insbesondere Trifluormethoxy, Trichlormethoxy und Pentafluorethoxy; C₁-C₄-Alkylthio wie vorstehend genannt, insbesondere Methylthio und Ethylthio und/oder partiell oder vollständig halogeniertes C₁-C₆-Alkylthio wie vorstehend genannt, insbesondere Difluormethylthio, Trifluormethylthio und Pentafluormethylthio;
R¹ ferner eine C₃-C₈-Cycloalkylgruppe wie Cyclopropyl, Cyclobutyl, Cyclopentyl, Cyclohexyl, Cycloheptyl und Cyclooctyl, insbesondere Cyclopropyl, Cyclopentyl und Cyclohexyl, wobei der Cyclus noch ein-bis dreimal durch C₁-C₄-Alkyl wie vorstehend genannt, insbesondere Methyl und Ethyl; oder Halogen wie Fluor, Chlor, Brom und Jod, insbesondere Fluor und Chlor, substituiert sein kann;
R¹ ferner die Phenylgruppe, welche noch einen bis drei der folgenden Reste tragen kann: Cyano; Nitro; Halogen wie Fluor, Chlor, Brom und Jod, insbesondere Fluor und Chlor; C₁-C₆-Alkyl wie vorstehend genannt, insbesondere Methyl, Ethyl und 1-Methylethyl; partiell oder vollständig halogeniertes C₁-C₆-Alkyl wie vorstehend genannt, insbesondere Trifluormethyl und Chlordifluormethyl; C₁-C₆-Alkoxy wie vorstehend genannt, insbesondere Methoxy und Ethoxy; partiell oder vollständig halogeniertes C₁-C₆-Alkoxy wie vorstehend genannt, insbesondere Trifluormethoxy, Trichlormethoxy und Pentafluorethoxy; C₁-C₄-Alkylthio wie vorstehend genannt, insbesondere Methylthio und Ethylthio und/oder partiell oder vollständig halogeniertes C₁-C₆-Alkylthio wie vorstehend genannt, insbesondere Difluormethylthio, Trifluormethylthio und Pentafluormethylthio;
R²
Wasserstoff;
C₁-C₆-Alkyl; bevorzugt C₁-C₄-Alkyl wie Methyl, Ethyl und tert. -Butyl, das einen bis drei der folgenden Substituenten tragen kann: Hydroxy, Halogen, wie Fluor, Chlor oder Brom, C₁-C₄-Alkoxy wie Methoxy und tert.-Butoxy, C₁-C₄-Alkylthio wie Methylthio und tert.-Butylthio;
C₃-C₈-Cycloalkyl, bevorzugt C₅-C₆-Cycloalkyl wie Cyclopentyl und Cyclohexyl, das ein- bis dreimal durch Halogen wie Fluor, Chlor und Brom, C₁-C₄-Alkyl wie Methyl und tert.-Butyl oder partiell oder vollständig halogeniertes C₁-C₄-Alkyl wie Fluormethyl, Trifluormethyl, Chlordifluormethyl, Pentafluorethyl und 2-Chlor-1,1,2-trifluorethyl substituiert sein kann;
R³
C₁-C₄-Alkoxy, wie vorstehend genannt, insbesondere Methoxy, Ethoxy und Isopropoxy;
verzweigtes oder unverzweigtes C₁-C₆-Alkyl, bevorzugt C₃-C₄-Alkyl, Isopropyl und tert.-Butyl, welches einen bis drei der folgenden Reste tragen kann: Halogen wie Fluor, Chlor und Brom, Cyano, C₁-C₄-alkoxy wie Methoxy und tert.-Butoxy, partiell oder vollständig halogeniertes C₁-C₄-Alkoxy wie Fluormethoxy, Trifluormethoxy, Trichlormethoxy, 2-Chlor-1,1,2-trifluorethoxy und Pentafluorethoxy, C₁-C₄-Alkylthio wie Methylthio und tert.-Butylthio, C₁-C₄-Halogenalkylthio wie Fluormethylthio, Trichlormethylthio, 2-Chlor-1,1,2-trifluorethylthio und Pentafluorethylthio, C₃-C₈-Cycloalkyl, insbesondere C₃-C₆-Cycloalkyl wie Cyclopropyl, Cyclopentyl und Cyclohexyl oder Phenyl, wobei der Phenylrest seinerseits bis zu drei der folgenden Gruppen tragen kann: Halogen wie Fluor, Chlor und Brom, Cyano, Nitro, C₁-C₄-Alkyl wie Methyl und tert.-Butyl, C₁-C₄-Halogenalkyl wie Fluormethyl, Trichlormethyl, 2-Chlor-1,1,2-trifluorethyl und Pentafluorethyl, C₁-C₄-Alkoxy wie Methoxy und tert.-Butoxy, partiell oder vollständig halogeniertes C₁-C₄-Alkoxy wie Trifluormethoxy, Trichlormethoxy, 2-Chlor-1,1,2-trifluorethoxy und Pentafluorethoxy, C₁-C₄-Alkylthio wie Methylthio und tert.-Butylthio oder C₁-C₄-Halogenalkylthio wie Trifluormethylthio, Trichlormethylthio, 2-Chlor-1,1,2-trifluorethylthio und Pentafluorethylthio;
R³ ferner C₃-C₈-Cycloalkyl, bevorzugt C₃-C₆-Cycloalkyl, insbesondere Cyclopropyl, Cyclopentyl und Cyclohexyl, das jeweils einen bis drei der folgenden reste tragen kann: Halogen wie Fluor, Chlor und Brom, Nitro, Cyano, C₁-C₆-Alkyl, bevorzugt C₁-C₄-Alkyl wie Methyl und tert.-Butyl, partiell oder vollständig halogeniertes C₁-C₆-Alkyl, bevorzugt C₁-C₄-Halogenalkyl wie Trifluormethyl, Trichlormethyl, 2-Chlor-1,1,2-trifluorethyl und Pentafluorethyl, C₁-C₄-Alkoxy wie Methoxy und tert.-Butoxy, oder partiell oder vollständig halogeniertes C₁-C₄-Alkoxy wie Trifluormethoxy, Trichlormethoxy, 2-Chlor-1,1,2-trifluorethoxy und Pentafluorethoxy;
eine C₃-C₆-Cycloalkenylgruppe, bevorzugt eine C₅-C₆-Cycloalkenylgruppe, die ein- bis dreimal durch Halogen wie Fluor, Chlor, Brom und Jod, insbesondere Fluor und Chlor, oder C₁-C₄-Alkyl wie Methyl, Ethyl und tert.-Butyl substituiert sein kann;
R³ ferner C₃-C₆-Alkenyl oder C₃-C₆-Alkinyl, bevorzugt C₃-C₄-Alkenyl oder C₃-C₅-Alkinyl wie 2-Propenyl, 2-Butenyl, 2-Propinyl, 1,1-Dimethyl-2-propinyl und 3-Butinyl, welches jeweils ein- bis dreifach durch Halogen wie Fluor, Chlor oder Brom und/oder einmal durch Phenyl substituiert sein kann, wobei der Phenylrest seinerseits eine bis drei der folgenden Substituenten tragen kann: Halogen, insbesondere Fluor und Chlor, Cyano, Nitro, C₁-C₄-Alkyl wie Methyl und tert.-Butyl, partiell oder vollständig halogeniertes C₁-C₄-Alkyl wie Fluormethyl, Trifluormethyl, Trichlormethyl, 2-Chlor-1,1,2-trifluorethyl und Pentafluorethyl, C₁-C₄-Alkoxy wie Methoxy und tert.-Butoxy, C₁-C₄-Halogenalkoxy wie Fluormethoxy, Trifluormethoxy, Trichlormethoxy, 2-Chlor-1,1,2-trifluorethoxy und Pentafluorethoxy, C₁-C₄-Alkylthio wie Methylthio und tert.-Butylthio, partiell oder vollständig halogeniertes C₁-C₄-Alkylthio wie Fluormethylthio, Trifluormethylthio, Trichlormethylthio, 2-Chlor-1,1,2-trifluorethylthio und Pentafluorethylthio;
R³ ferner die Phenylgruppe welche noch einen bis drei der folgenden Reste tragen kann: Cyano; Nitro; Halogen wie Fluor, Chlor, Brom und Jod, insbesondere Fluor und Chlor; C₁-C₆-Alkyl wie vorstehend genannt, insbesondere Methyl, Ethyl und 1-Methylethyl; partiell oder vollständig halogeniertes C₁-C₆-Alkyl wie vorstehend genannt, insbesondere Trifluormethyl und Chlordifluormethyl; C₁-C₆-Alkoxy wie vorstehend genannt, insbesondere Methoxy und Ethoxy; partiell oder vollständig halogeniertes C₁-C₆-Alkoxy wie vorstehend genannt, insbesondere Trifluormethoxy, Trichlormethoxy und Pentafluorethoxy; C₁-C₄-Alkylthio wie vorstehend genannt, insbesondere Methylthio und Ethylthio und/oder partiell oder vollständig halogeniertes C₁-C₆-Alkylthio wie vorstehend genannt, insbesondere Difluormethylthio, Trifluormethylthio und Pentafluorethylthio.

Die Verbindungen Ia oder Ib, z.B./bzw. die diese Verbindungen enthaltenden herbiziden Mittel können in Kulturen wie Weizen, Reis, Mais, Soja und Baumwolle Unkräuter und Schadgräser sehr gut bekämpfen, ohne die Kulturpflanzen zu schädigen.

Die Verbindungen Ia oder Ib bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen Ia oder Ib eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Methanol, Ethanol, Propanol, Butanol, Cyclohexanol, Cyclohexanon oder stark polare Lösungsmittel, wie N-Methylpyrrolidon oder Wasser in Betracht.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Formulierungen enthalten im allgemeinen zwischen 0,01 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum), eingesetzt.

Die erfindungsgemäßen Verbindungen I können beispielsweise wie folgt formuliert werden:
I. 20 Gewichtsteile der Verbindung Nr. 2.009 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.
II. 20 Gewichtsteile der Verbindung Nr. 2.009 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Ein-gießen und feines Verteilen der Lösung in 100 000 Gewichtsteilen Wasser erhält man eine wäßrige Dispersion, die 0,02 Gew.% des Wirkstoffs enthält.
III. 20 Gewichtsteile des Wirkstoffs Nr. 2.009 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinus besteht. Durch Eingießen und feines Verteilen der Lösung in 100 000 Gewichtsteilen Wasser erhält man eine wäßrige Dispersion, die 0,02 Gew.% des Wirkstoffs enthält.
IV. 20 Gewichtsteile des Wirkstoffs Nr. 2.009 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.% des Wirkstoffs enthält.
V. 3 Gewichtsteile des Wirkstoffs Nr. 2.009 werden mit 97 Gewicht steilen feinteiligem Kaolin vermischt. Man erhält auf diese Weise ein Stäubemittel, das 3 Gew.% des Wirkstoffs enthält.
VI. 20 Gewichtsteile des Wirkstoffs Nr. 2.009 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0, vorzugsweise 0,01 bis 2,0 kg/ha aktive Substanz (a.S.).

In Anbetracht der Vielseitigkeit der Applikationsmethoden können die Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicaco sativa, Musa spp., Nicotiana tabacum (N.rustica), Olea europaea, Oryza sativa , Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Zur Verbreiterung des Wirkungsspektrums und zur Erzielung synergistischer Effekte können die Cyanoisoxazole Ia bzw. Ib mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen Ia bzw. Ib allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Herstellungsbeispiele

### 4-Carboxamido-3-isopropyl-isoxazol-5-carbonsäurecyclopropylamid

9,9 g 5-Cyclopropylaminocarbonyl-3-isopropyl-isoxazol-4-carbonsäure und 9,9 g Thionylchlorid werden in 150 ml Toluol 1,5 Stunden zum Rückfluß erhitzt. Zur Abtrennung überschüssigen Thionylchlorids engt man zur Trockene ein, nimmt anschließend den Rückstand erneut in 50 ml Toluol auf und tropft diese Lösung gleichzeitig mit 5,7 ml einer 25 %igen wäßrigen Ammoniaklösung bei Raumtemperatur in eine Vorlage aus 100 ml Toluol. Man läßt 14 Stunden rühren, gibt dann Wasser zu und saugt das ausgefallene Produkt ab.

Ausbeute: 2,1 g, Fp. 152 - 156°C.

In gleicher Weise wurden die in Tabelle 1 wiedergegebenen Zwischenprodukte III synthetisiert:

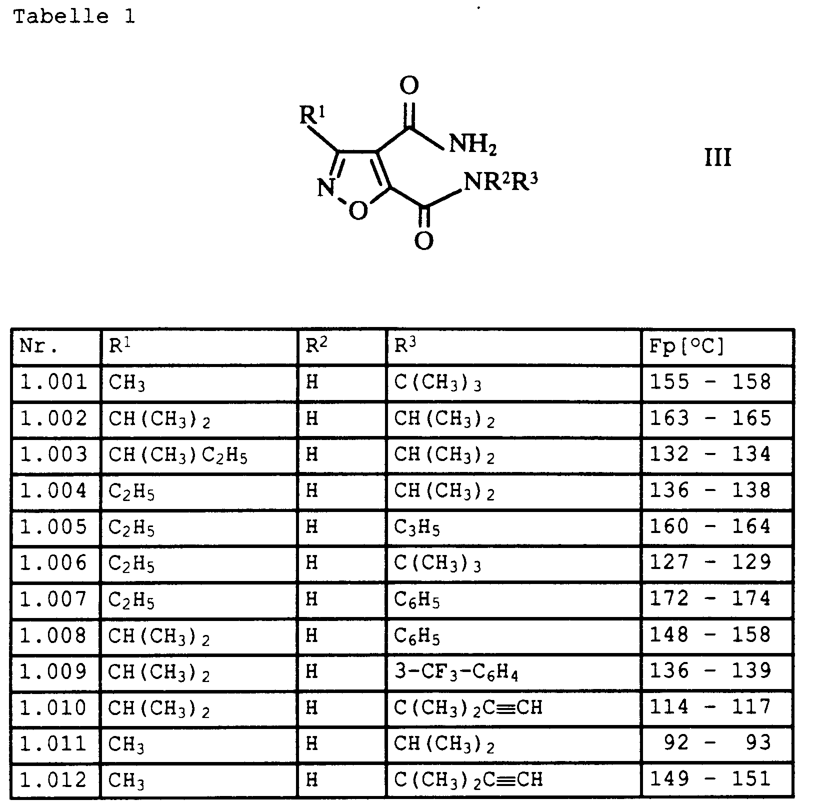

### 4-Cyano-3-isopropyl-isoxazol-5-carbonsäurecyclopropylamid

Zu einer Lösung von 2,1 g 4-Carboxamido-3-isopropyl-isoxazol-5-carbonsäurecyclopropylamid und 3,7 g Kaliumcarbonat in 150 ml Acetonitril werden bei 60°C 2,7 g Phosphoroxytrichlorid zugetropft. Anschließend rührt man 4 Stunden bei 70°C nach. Zur Aufarbeitung gießt man die Lösung auf 200 ml Eiswasser, extrahiert mit Essigsäureethylester und wäscht die organische Phase dann mit wäßriger Natriumhydrogencarbonat-Lösung und wäßriger Natriumchlorid-Lösung. Die organische Phase wird über Magnesiumsulfat getrocknet und vom Lösungsmittel befreit.
Ausbeute: 1,9 g, Fp.: 107 - 108°C (Bsp. Nr. 2.009)

In analoger Weise wurden die in nachstehender Tabelle 2 aufgeführten Verbindungen Ia erhalten:

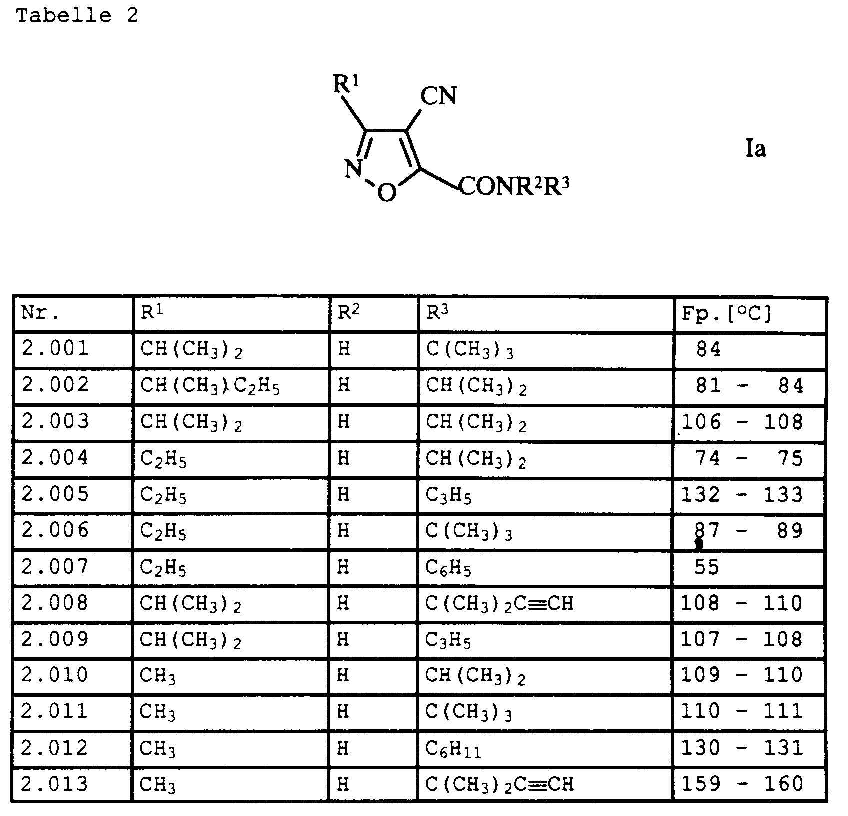

### 3-Isopropyl-isoxazol-4,5-dicarbonsäure-4-methylester

40 g 3-Isopropyl-isoxazol-4,5-dicarbonsäuredimethylester werden in 200 ml Methanol gelöst. Bei 0°C tropft man hierzu eine Lösung von 7,05 g Natriumhydroxid in 50 ml Wasser. Man läßt 14 Stunden rühren und dabei auf Raumtemperatur aufwärmen. Die Reaktionslösung wird mit Wasser verdünnt und zweimal mit Essigsäureethylester extrahiert. Anschließend säuert man die alkalische Lösung mit konz. Salzsäure an, extrahiert mehrmals mit Essigsäureethylester, trocknet die organische Phase und engt ein. Man erhält 33,7 g eines Öls, welches ohne weitere Reinigung eingesetzt wird.

### 5-Carboxamido-3-isopropyl-isoxazol-4-carbonsäuremethylester

33,6 g 3-Isopropyl-isoxazol-4,5-dicarbonsäure-4-methylester und 37,5 g Thionylchlorid werden in 150 ml Toluol zwei Stunden zum Rückfluß erhitzt. Anschließend engt man im Vakuum zur Trockene ein.

26,8 g des erhaltenen Öls werden in 75 ml Toluol gelöst und gleichzeitig mit einer Lösung von konz. Ammoniak (161 ml) in eine Vorlage aus 100 ml Toluol bei 0 - 10°C getropft. Man läßt 14 Stunden bei Raumtemperatur rühren, gibt dann Wasser zu und saugt den entstandenen Niederschlag ab.

Ausbeute 15,6 g, Fp. 229 - 231°C.

### 5-Carboxamido-3-isopropyl-isoxazol-4-carbonsäure

Zu einer Lösung aus 15,6 g 5-Carboxamido-3-isopropyl-isoxazol-4-carbonsäuremethylester in 200 ml Methanol wird bei 0 - 10°C eine Lösung aus 2,9 g Natriumhydroxid in 50 ml Wasser getropft. Man läßt 14 Stunden rühren, verdünnt mit Wasser, extrahiert zweimal mit Essigsäureethylester und säuert die wäßrige Lösung an. Das ausgefallene Öl kann in Essigsäuremethylester aufgenommen werden. Nach einengen der organischen Phase erhält man das gewünschte Produkt.

Ausbeute 9,1 g, Fp. 150 - 152°C.

### 5-Cyano-3-isopropyl-isoxazol-4-carbonsäurecyclopropylamid und 5-Carboxamido-3-isopropyl-isoxazol-4-carbonsäurecyclopropylamid

9,1 g Carboxamido-3-isopropyl-isoxazol-4-carbonsäure und 21,9 g Thionylchlorid werden in 200 ml Toluol zwei Stunden zum Rückfluß erhitzt. Nach einengen zur Trockene erhält man 10,6 g eines Öls, welches 5-Cyano-3-isopropyl-isoxazol-4-carbonsäurechlorid und 5-Carboxamido-3-isopropyl-4-carbonsäurechlorid enthält.

3,5 g dieses Öls werden in 150 ml Toluol vorgelegt und bei 0 - 10°C mit 2,0 g Cyclopropylamin versetzt. Man läßt 14 Stunden bei Raumtemperatur rühren, verdünnt dann mit Wasser, trennt die organische Phase ab und extrahiert diese mit wäßriger Natriumhydrogencarbonat-Lösung. Man engt die organische Phase zur Trockene ein und isoliert nach Chromatographie an Kieselgel (Eluent: Cyclohexan/Essigsäureethylester) 1,8 g 5-Cyano-3-isopropyl-isoxazol-4-carbonsäurecyclopropylamid, Fp. 105 - 108°C und 0,6 g 5-Carboxamido-3-isopropyl-isoxazol-4-carbonsäurecyclopropylamid, Fp. 130 - 134°C.

Die 5-Carboxamido-Verbindung läßt sich durch Behandlung mit Thionylchlorid in Toluol bei Rückfluß-Temperatur in die 5-Cyano-Verbindung überführen.

In gleicher Weise wurden die Zwischenprodukte V (Tabelle 3) und die Endprodukte Ib (Tabelle 4) synthetisiert.

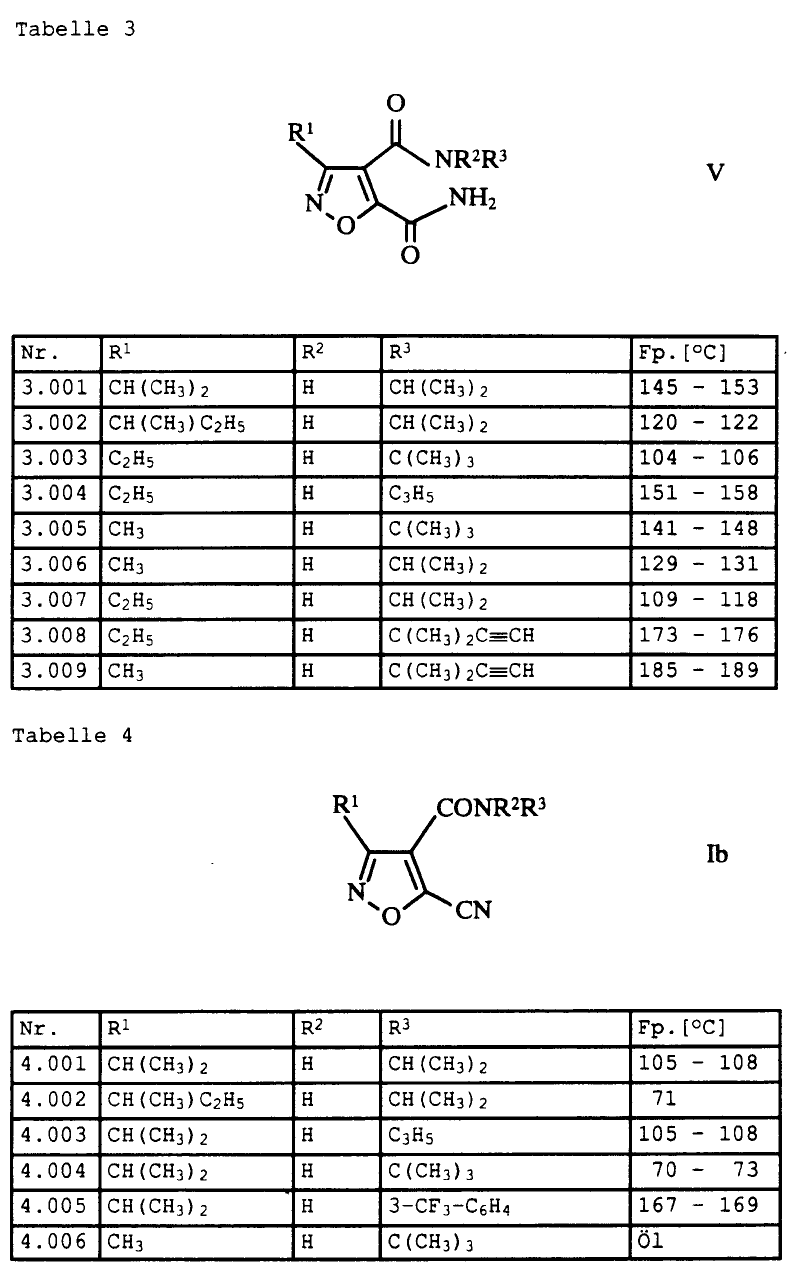

### Anwendungsbeispiele

Die herbizide Wirkung der Cyanoisoxazole der Formel Ia bzw. Ib ließ sich durch Gewächshausversuche zeigen:
Als Kulturgefäße dienten Plastikblumentöpfe mit lehmigem Sand mit etwa 3,0% Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt.

Die Pflanzen wurden artenspezifisch bei Temperaturen von 10 - 25 °C bzw. 20 - 35 °C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die cyano-substituierten Verbindungen Ia bzw. Ib zeigten sehr gute herbizide Wirkung. Gleichzeitig weisen sie ein überraschend vorteilhaftes Abbauverhalten im Boden auf. So wird beispielsweise für Verbindung Nr. 2.009 eine Halbwertszeit DT₅₀ von nur 1 - 2 Wochen ermittelt. Dagegen werden strukturell ähnliche Verbindungen des Standes der Technik, z.B. Vergleichssubstanz A, bekannt aus EP-A 337 263, deutlich langsamer abgebaut. Die Metabolisierungsversuche mit A ergaben eine DT₅₀ von mehr als 10 Wochen.

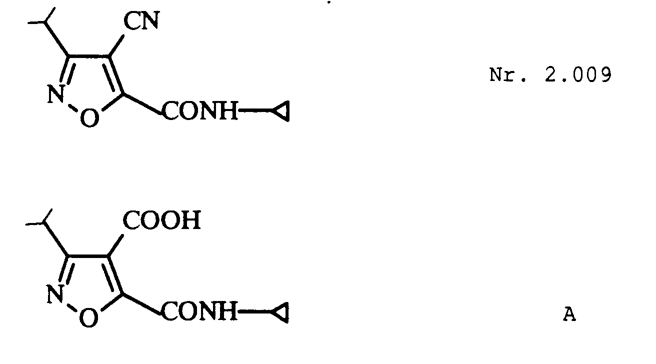

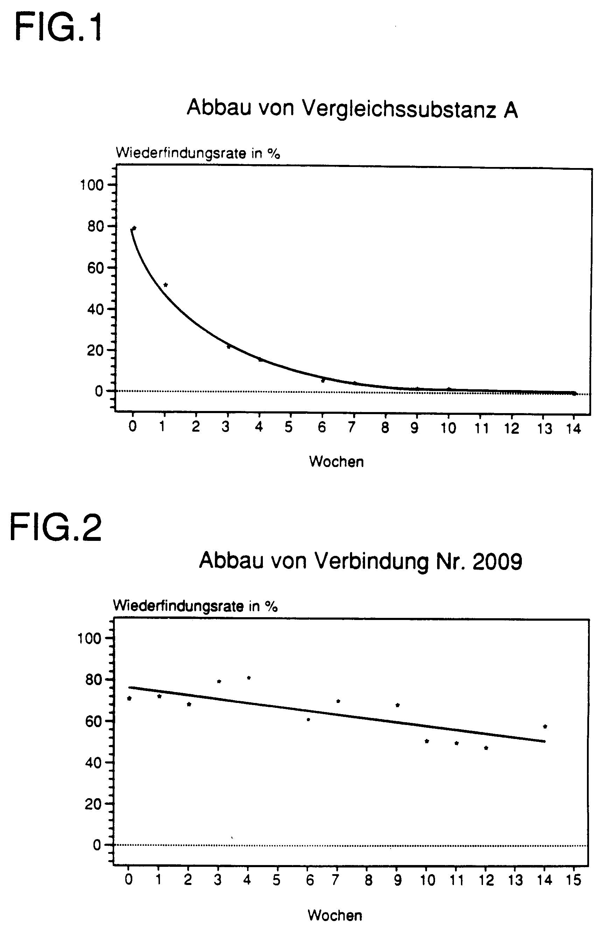

Zur Ermittlung des Abbauverhaltens wurden die Verbindungen zu einer Mischung aus 70 % Cyclohexan, 20 % Nekanil® NL (ethoxyliertes Isooctylphenol) und 10 % Emulphor® EC (ethoxyliertes Rizinusöl) gegeben, so daß der Wirkstoffgehalt bei 100 g/l lag. Von dem so formulierten Wirkstoff wurden 113,4 µl in 100 ml bidest. Wasser gelöst und davon jeweils 1 ml zu einer definierten, in Plastikbeutel eingewogenen Menge (Einwaage pro Beutel bezog sich auf jeweils 100 g Trockengewicht des Bodens) von biologisch aktivem Feldboden (Eigenschaften: Lehmiger Sand; pH 6,1; Humus 1,2 %, KAK 4,8 mval Ba/100 g Boden) gegeben. Dies ergibt eine Aufwandmenge von 0,6 kg a.S./ha. Durch Zusatz einer berechneten Wassermenge wurde der Boden auf 40 % seiner max. Wasserkapazität eingestellt und die so präparierten Proben aus aktiver Substanz, Feldboden und Wasser gut durchmischt und bei einer Temperatur von 20°C aufbewahrt. Während der Versuchsdauer (30.09.93 - 06.01.94) wurden Bodenproben mit Methanol extrahiert und die Metabolisierungsrate über HPLC ermittelt. Die Versuchsergebnisse sind den Figuren 1 und 2 zu entnehmen.

## Patentansprüche

1. Cyanoisoxazole der allgemeinen Formeln Ia und Ib 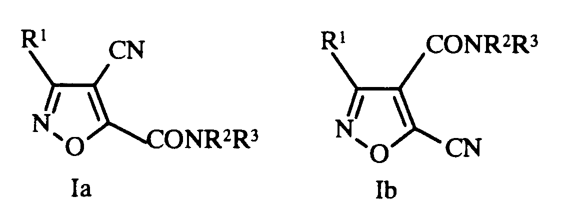 in denen
R¹
Wasserstoff;
C₁-C₆-Alkyl, welches ein bis fünf Halogenatome und/oder einen Cyanorest und/oder bis zu zwei der folgenden Reste tragen kann: C₁-C₄-Alkoxy, C₁-C₄-Halogenalkoxy, C₁-C₄-Alkylthio, C₁-C₄-Halogenalkylthio, C₃-C₆-Cycloalkyl, welches wiederum durch ein bis drei Halogen- oder C₁-C₄-Alkylreste substituiert sein kann oder Phenyl, welches noch einen bis drei der folgenden Reste tragen kann: Cyano, Halogen, Nitro, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio;
C₃-C₈-Cycloalkyl, welches ein- bis dreimal durch C₁-C₄-Alkyl und/oder Halogen substituiert sein kann;
Phenyl, welches noch einen bis drei der folgenden Reste tragen kann: Cyano, Nitro, Halogen, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio;
R²
Wasserstoff;
C₁-C₆-Alkyl, das einen bis drei der folgenden Substituenten tragen kann: Hydroxy, Halogen, C₁-C₄-Alkoxy und/oder C₁-C₄-Alkylthio;
C₃-C₈-Cycloalkyl, das ein- bis dreimal durch Halogen, C₁-C₄-Alkyl und/oder C₁-C₄-Halogenlalkyl substituiert sein kann;
R³
eine C₁-C₄-Alkoxygruppe;
eine C₁-C₆-Alkylgruppe, die einen bis drei der folgenden Reste tragen kann: Halogen, Cyano, C₁-C₄-Alkoxy, C₁-C₄-Halogenalkoxy, C₁-C₄-Alkylthio, C₁-C₄-Halogenalkylthio, C₃-C₈-Cycloalkyl oder Phenyl, wobei der Phenylring seinerseits einen bis drei der folgenden Reste tragen kann: Halogen, Cyano, Nitro, C₁-C₄-Alkyl, C₁-C₄-Halogenalkyl, C₁-C₄-Alkoxy, C₁-C₄-Halogenalkoxy, C₁-C₄-Alkylthio und/oder C₁-C₄-Halogenalkylthio;
eine C₃-C₆-Cycloalkenylgruppe, die ein- bis dreimal durch Halogen oder C₁-C₄-Alkyl substituiert sein kann;
eine C₃-C₈-Cycloalkylgruppe, die einen bis drei der folgenden Reste tragen kann: Halogen, Nitro, Cyano, C₁-C₆-Alkyl, C₁-C₆-Halogenalkyl, C₁-C₄-Alkoxy und/oder C₁-C₄-Halogenalkoxy;
eine C₃-C₆-Alkenyl- oder C₃-C₆-Alkinylgruppe, die jeweils einbis dreimal durch Halogen und/oder einmal durch Phenyl substituiert sein können, wobei der Phenylrest seinerseits eine bis drei der folgenden Gruppen tragen kann: C₁-C₄-Alkyl, C₁-C₄-Halogenalkyl, C₁-C₄-Alkoxy, C₁-C₄-Halogenalkoxy, C₁-C₄-Alkylthio, C₁C₄-Halogenalkylthio, Halogen, Cyano oder Nitro;
die Phenylgruppe, welche noch einen bis drei der folgenden Reste tragen kann: Cyano, Nitro, Halogen, C₁-C₆-Alkyl, C₁-C₆-Halogenalkyl, C₁-C₆-Alkoxy, C₁-C₆-Halogenalkoxy, C₁-C₆-Alkylthio und/oder C₁-C₆-Halogenalkylthio.

2. Verfahren zur Synthese von Verbindungen der allgemeinen Formel Ia, dadurch gekennzeichnet, daß man ein Isoxazol-4,5-dicarbonsäure-monoamid der Formel II 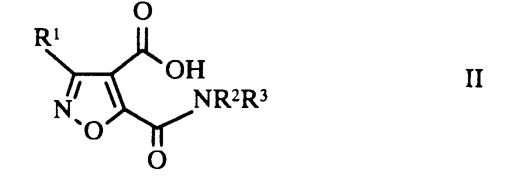 in der R¹, R² und R³ die in Anspruch 1 genannten Bedeutungen haben, amidiert und die Diamide III 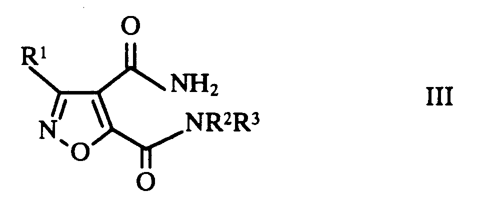 dann zu den Nitrilen Ia dehydratisiert.

3. Verfahren zur Synthese von Verbindungen der allgemeinen Formel Ib, dadurch gekennzeichnet, daß man ein Isoxazol-4,5-dicarbonsäure-monoamid der Formel IV 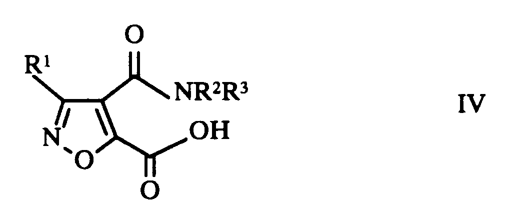 in der R¹, R² und R³ die in Anspruch 1 genannten Bedeutungen haben, amidiert und die Diamide V 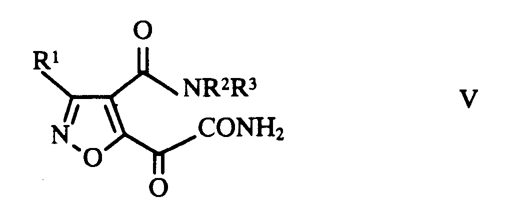 dann zu den Nitrilen Ib dehydratisiert.

4. Verfahren zur Synthese von Verbindungen der allgemeinen Formel Ib, dadurch gekennzeichnet, daß man einen Isoxazol-4,5-dicarbonsäure-ester der Formel VI 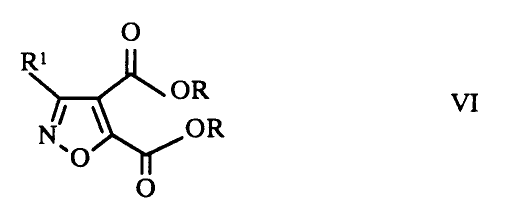 in der R¹ die in Anspruch 1 genannte Bedeutung hat und R für einen C₁-C₄-Alkylrest steht, in die Monocarbonsäure VII überführt, 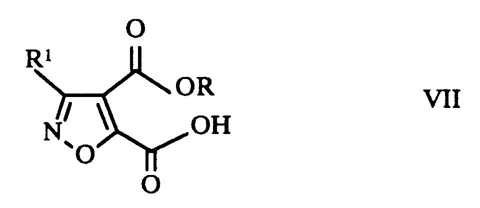 diese zum Amid VIII 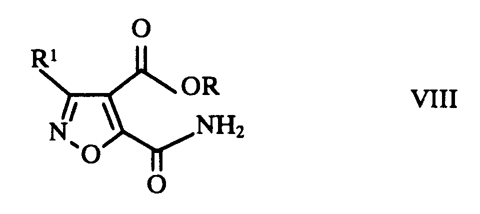 umsetzt, anschließend nach einem weiteren Hydrolyseschritt die Säure IX 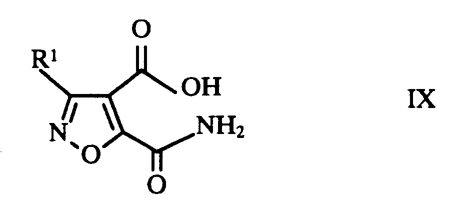 amidiert und das Diamid V 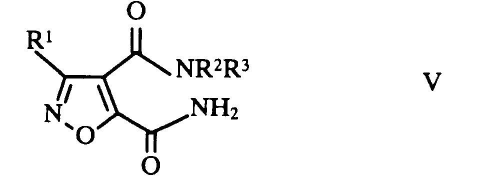 zum Nitril Ib dehydratisiert.

5. Mittel, enthaltend inerte Trägerstoffe und eine herbizid wirksame Menge mindestens eines Cyanoisoxazols der Formel Ia bzw. 1b gemäß Anspruch 1.

6. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Cyanoisoxazols der Formel Ia bzw. Ib gemäß Anspruch 1 behandelt.

## Claims

1. A cyanoisoxazole of the general formula Ia or Ib 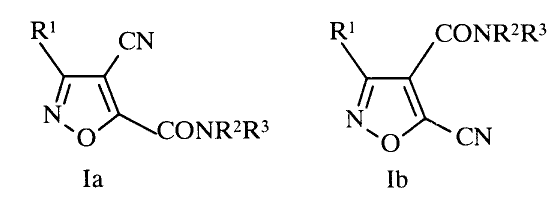 where
R¹ is
hydrogen;
C₁-C₆-alkyl which can carry one to five halogen atoms and/or one cyano radical and/or up to two of the following radicals: C₁-C₄-alkoxy, C₁-C₄-haloalkoxy, C₁-C₄-alkylthio, C₁-C₄-haloalkylthio, C₃-C₆-cycloalkyl, which in turn can be substituted by one to three halogen or C₁-C₄-alkyl radicals or phenyl which can additionally carry one to three of the following radicals: cyano, halogen, nitro, C₁-C₆-alkyl, C₁-C₆-haloalkyl, C₁-C₆-alkoxy, C₁-C₆-haloalkoxy, C₁-C₆-alkylthio, C₁-C₆-haloalkylthio;
C₃-C₈-cycloalkyl which can be substituted one to three times by C₁-C₄-alkyl and/or halogen;
phenyl which can additionally carry one to three of the following radicals: cyano, nitro, halogen, C₁-C₆-alkyl, C₁-C₆-haloalkyl, C₁-C₆-alkoxy, C₁-C₆-haloalkoxy, C₁-C₆-alkylthio, C₁-C₆-haloalkylthio;
R² is
hydrogen;
C₁-C₆-alkyl which can carry one to three of the following substituents: hydroxyl, halogen, C₁-C₄-alkoxy and/or C₁-C₄-alkylthio;
C₃-C₈-cycloalkyl which can be substituted one to three times by halogen, C₁-C₄-alkyl and/or C₁-C₄-haloalkyl;
R³ is
a C₁-C₄-alkoxy group;
a C₁-C₆-alkyl group which can carry one to three of the following radicals: halogen, cyano, C₁-C₄-alkoxy, C₁-C₄-haloalkoxy, C₁-C₄-alkylthio, C₁-C₄-aloalkylthio, C₃-C₈-cycloalkyl or phenyl, it being possible for the phenyl ring in turn to carry one to three of the following radicals: halogen, cyano, nitro, C₁-C₄-alkyl, C₁-C₄-haloalkyl, C₁-C₄-alkoxy, C₁-C₄-haloalkoxy, C₁-C₄-alkylthio and/or C₁-C₄-haloalkylthio;
a C₃-C₆-cycloalkenyl group which can be substituted one to three times by halogen or C₁-C₄-alkyl;
a C₃-C₈-cycloalkyl group which can carry one to three of the following radicals: halogen, nitro, cyano, C₁-C₆-alkyl, C₁-C₆-haloalkyl, C₁-C₄-alkoxy and/or C₁-C₄-haloalkoxy;
a C₃-C₆-alkenyl or C₃-C₆-alkynyl group which in each case can be substituted one to three times by halogen and/or once by phenyl, it being possible for the phenyl radical in turn to carry one to three of the following groups: C₁-C₄-alkyl, C₁-C₄-haloalkyl, C₁-C₄-alkoxy, C₁-C₄-haloalkoxy, C₁-C₄-alkylthio, C₁-C₄-haloalkylthio, halogen, cyano or nitro;
the phenyl group, which can additionally carry one to three of the following radicals: cyano, nitro, halogen, C₁-C₆-alkyl, C₁-C₆-haloalkyl, C₁-C₆-alkoxy, C₁-C₆-haloalkoxy, C₁-C₆-alkylthio and/or C₁-C₆-haloalkylthio.

2. A process for synthesizing compounds of the general formula Ia, which comprises amidating an isoxazole-4,5-dicarboxylic acid monoamide of the formula II 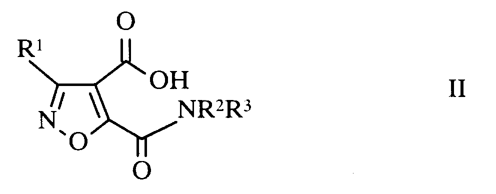 where R¹, R² and R³ have the meanings mentioned in claim 1, and then dehydrating the diamide III 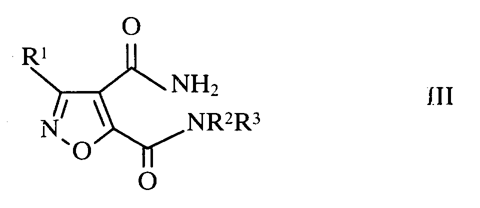 to give the nitrile Ia.

3. A process for synthesizing compounds of the general formula Ib, which comprises amidating an isoxazole-4,5-dicarboxylic acid monoamide of the formula IV 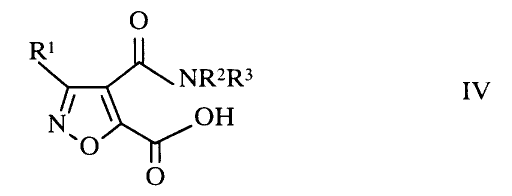 where R¹, R² and R³ have the meanings mentioned in claim 1, and then dehydrating the diamide V 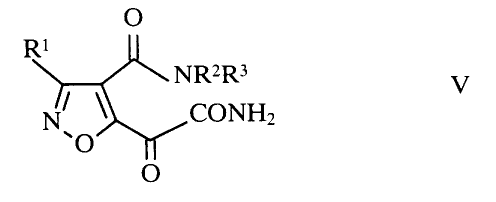 to give the nitrile Ib.

4. A process for synthesizing compounds of the general formula Ib, which comprises converting an isoxazole-4,5-dicarboxylic acid ester of the formula VI 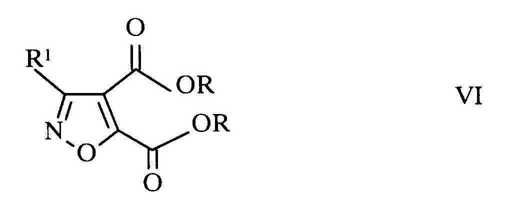 where R¹ has the meaning mentioned in claim 1 and R is a C₁-C₄-alkyl radical, to the monocarboxylic acid VII 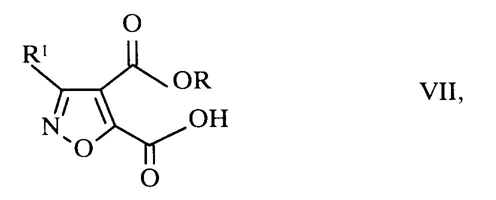 reacting this to give the amide VIII 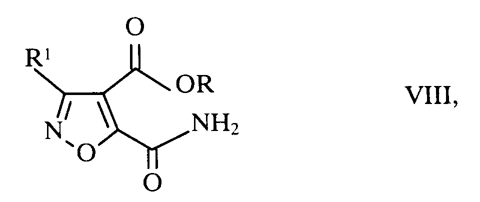 then after a further hydrolysis step amidating the acid IX 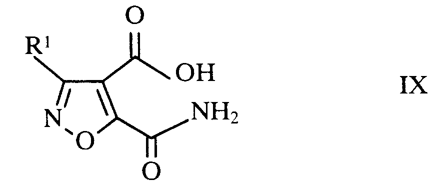 and dehydrating the diamide V 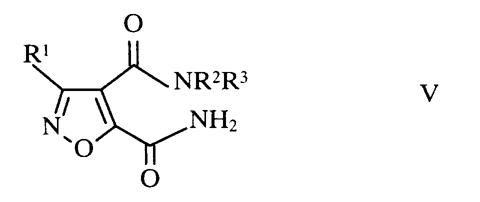 to give the nitrile Ib.

5. A composition containing inert carriers and a herbicidally active amount of at least one cyanoisoxazole of the formula Ia or Ib as claimed in claim 1.

6. A method for controlling undesired plant growth, which comprises treating the undesired plants and/or their habitat with a herbicidally active amount of a cyanoisoxazole of the formula Ia or Ib as claimed in claim 1.

## Revendications

1. Cyanoisoxazoles répondant aux formules générales Ia et Ib 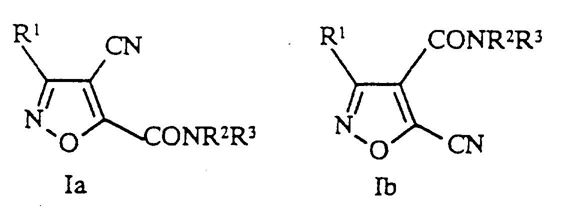 dans lesquelles
R¹ représente
l'hydrogène ;
un groupe alkyle en C1-C6 qui peut porter un à cinq atomes d'halogènes et/ou un groupe cyano et/ou jusqu'à deux des substituants suivants : alcoxy en C1-C4, halogénoalcoxy en C1-C4, alkylthio en C1-C4, halogénoalkylthio en C1-C4, cycloalkyle en C3-C6, lequel peut lui-même porter un à trois substituants halogéno ou alkyle en C1-C4, ou phényle, lequel peut porter un à trois des substituants suivants : cyano, halogéno, nitro, alkyle en C1-C6, halogénoalkyle en C1-C6, alcoxy en C1-C6, halogénoalcoxy en C1-C6, alkylthio en C1-C6, halogénoalkylthio en C1-C6 ;
un groupe cycloalkyle en C3-C8 qui peut porter un à trois substituants alkyle en C1-C4 et/ou halogéno ;
un groupe phényle qui peut porter un à trois des substituants suivants : cyano, nitro, halogéno, alkyle en C1-C6, halogénoalkyle en C1-C6, alcoxy en C1-C6, halogénoalcoxy en C1-C6, alkylthio en C1-C6, halogénoalkylthio en C1-C6 ;
R² représente
l'hydrogène ;
un groupe alkyle en C1-C6 qui peut porter un à trois des substituants suivants : hydroxy, halogéno, alcoxy en C1-C4 et/ou alkylthio en C1-C4 ;
un groupe cycloalkyle en C3-C8 qui peut porter un à trois substituants halogéno, alkyle en C1-C4 et/ou halogénoalkyle en C1-C4 ;
R³ représente
un groupe alcoxy en C1-C4 ;
un groupe alkyle en C1-C6 qui peut porter un à trois des substituants suivants : halogéno, cyano, alcoxy en C1-C4, halogénoalcoxy en C1-C4, alkylthio en C1-C4, halogénoalkylthio en C1-C4, cycloalkyle en C3-C8 ou phényle, lequel peut porter lui-même dans le cycle un à trois des substituants suivants : halogéno, cyano, nitro, alkyle en C1-C4, halogénoalkyle en C1-C4, alcoxy en C1-C4, halogénoalcoxy en C1-C4, alkylthio en C1-C4 et/ou halogénoalkylthio en C1-C4 ;
un groupe cycloalcényle en C3-C6 qui peut porter un à trois substituants halogéno ou alkyle en C1-C4 ;
un groupe cycloalkyle en C3-C8 qui peut porter un à trois des substituants suivants : halogéno, nitro, cyano, alkyle en C1-C6, halogénoalkyle en C1-C6, alcoxy en C1-C4 et/ou halogénoalcoxy en C1-C4 ;
un groupe alcényle en C3-C6 ou alcynyle en C3-C6, chacun d'eux pouvant porter un à trois substituants halogéno et/ou un substituant phényle, ce dernier pouvant lui-même porter un à trois des substituants suivants : alkyle en C1-C4, halogénoalkyle en C1-C4, alcoxy en C1-C4, halogénoalcoxy en C1-C4, alkylthio en C1-C4, halogénoalkylthio en C1-C4, halogéno, cyano ou nitro ;
le groupe phényle qui peut encore porter un a trois des substituants suivants : cyano, nitro, halogéno, alkyle en C1-C6, halogénoalkyle en C1-C6, alcoxy en C1-C6, halogénoalcoxy en C1-C6, alkylthio en C1-C6 et/ou halogénoalkylthio en C1-C6.

2. Procédé pour la synthèse des composés de formule générale Ia, caractérisé par le fait que l'on amide un monoamide d'acide isoxazole-4,5-dicarboxylique de formule II 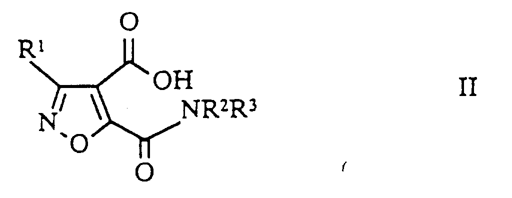 dans laquelle R¹, R² et R³ ont les significations indiquées dans la revendication 1, ce qui donne les diamides III 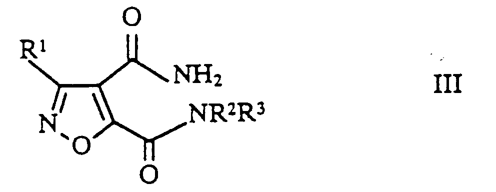 qu'on déshydrate ensuite en nitriles Ia.

3. Procédé pour la synthèse des composés de formule générale Ib, caractérisé par le fait que l'on amide un monoamide d'acide isoxazole-4,5-dicarboxylique de formule IV 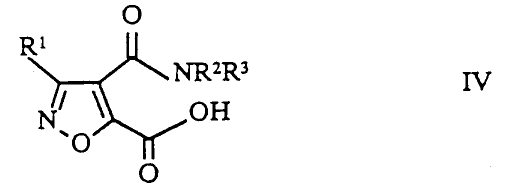 dans laquelle R¹ R² et R³ ont les significations indiquées dans la revendication 1, ce qui donne les diamides V 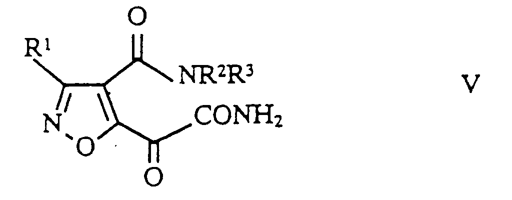 qu'on convertit en les nitriles Ib par déshydratation.

4. Procédé pour la synthèse des composés de formule général Ib, caractérisé par le fait que l'on convertit un ester d'acide isoxazole-4,5-dicarboxylique de formule VI 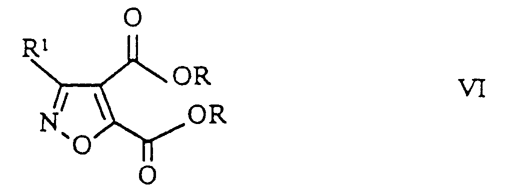 dans laquelle R¹ a les significations indiquées dans la revendication 1 et R représente un groupe alkyle en C1-C4, en l'acide monocarboxylique VII 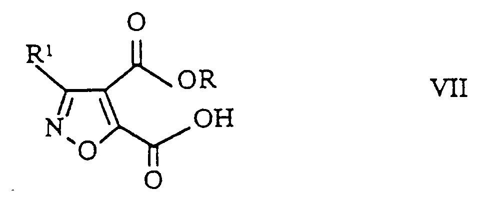 qu'on convertit alors en l'amide VIII 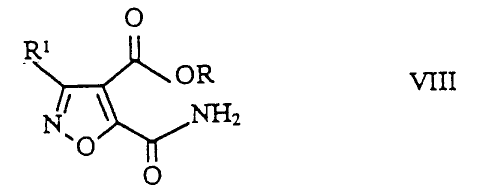 lequel est converti par hydrolyse en l'acide IX 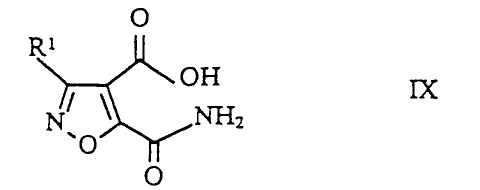 qu'on amide, ce qui donne le diamide V 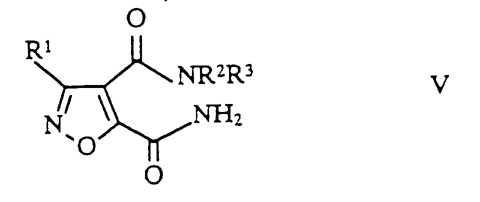 qu'on convertit en le nitrile Ib par déshydratation.

5. Produit contenant des véhicules inertes et une quantité herbicide efficace d'au moins un cyanoisoxazole de formule Ia ou Ib selon la revendication 1.

6. Procédé pour combattre les croissances de végétaux indésirables, caractérisé par le fait que l'on traite les végétaux indésirables et/ou leur habitat par une quantité herbicide efficace d'un cyanoisoxazole de formule Ia ou Ib selon la revendication 1.
